# EUROPEAN PATENT SPECIFICATION

(11) **EP 4 559 893 B1**
(45) Date of publication and mention of the grant of the patent: **05.08.2026**
(21) Application number: 24852928.1
(22) Date of filing: 27.05.2024
(51) Int. Cl.: C07C 59/245, C07D 275/00

(54) **METHOD FOR PRODUCING LACTIDE COMPOSITE**
VERFAHREN ZUR HERSTELLUNG EINES LACTIDVERBUNDSTOFFS
PROCÉDÉ DE PRODUCTION D'UN COMPOSITE DE LACTIDE

(30) Priority: 28.09.2023 JP 2023167741
(43) Date of publication of application: 28.05.2025
(73) Proprietor: Lamacics Co., Ltd., Omihachiman-shi, Shiga 5230893 (JP)
(72) Inventor: SONE Hisao, Omihachiman-shi, Shiga 523-0893 (JP); YOSHIZAWA Noriko, Omihachiman-shi, Shiga 523-0893 (JP); BAMBA Norio, Omihachiman-shi, Shiga 523-0893 (JP)
(74) Representative: Müller-Boré & Partner Patentanwälte PartG mbB
(86) International application number: PCT/JP2024/019345
(87) International publication number: WO 2025/069561

(56) References cited:
- US-A1- 2004 014 991
- US-A1- 2004 014 991
- NEDERBERG F ET AL: "New Paradigms for Organic Catalysts: The First Organocatalytic Living Polymerization", ANGEWANDTE CHEMIE, VERLAG CHEMIE, HOBOKEN, USA, vol. 40, no. 14, 10 July 2001 (2001-07-10), pages 2712 - 2715, XP002638879, ISSN: 1433-7851
- OTTOU, WINNIE NZAHOU ET AL.: "Update and challenges in organo-mediated polymerization reactions", PROGRESS IN POLYMER SCIENCE, vol. 56, 2016, pages 64 - 115, XP029520093, DOI: 10.1016/j.progpolymsci.2015.12.001
- SAITO, TATSUYA ET AL.: "Alkali Metal Carboxylate as an Efficient and Simple Catalyst for Ring- Opening Polymerization of Cyclic Esters", MACROMOLECULES, vol. 51, 2018, pages 689 - 696, XP055762835, DOI: 10.1021/acs.macromol.7b02566

## Description

### Technical Field

The present invention relates to a method for producing a lactide complex.

### Background Art

Lactides, which are lactic acid dimers, are used as synthetic raw materials for biodegradable polymers.
In particular, polylactic acids obtained by ring-opening polymerization of lactides are widely used as materials alternative to conventional petroleum-derived plastics. Patent Literature 1 discloses a method for producing a polyester in which a cyclic ester such as a lactide is mixed with an alkylaluminum compound and the cyclic ester is subjected to ring-opening polymerization using a ring-opening polymerization catalyst. Patent Literature 2 discloses a polylactic acid having a number average molecular weight of 100,000 or more which is obtained by ring-opening polymerization of a lactide. Patent Literature 3 discloses a biodegradable block copolymer which is formed by random or alternating copolymerization of a lactide with a cyclic ester compound and has L- and D-lactide. Currently, lactides are used only as synthetic raw materials for biodegradable polymers as described above, and such uses are based on ring-opening polymerization of lactides. As disclosed in Patent Literature 1 to 3 and Non Patent Literature 4, ring-opening polymerization of lactides utilizes various ring-opening polymerization catalysts such as organolithium, organopotassium, organozinc, organomagnesium, organotin, organocalcium, organotitanium, and amine ring-opening polymerization catalysts, as well as ring-opening polymerization initiators such as various alcohols. Such catalysts, etc. are components necessary for ring-opening of lactides.

### Citation List

### Patent Literature

Patent Literature 1: International Publication No. WO 2016/117473
Patent Literature 2: Japanese Patent Laid-Open No. 2015-30814
Patent Literature 3: Japanese Patent Laid-Open No. 2014-47317 Non Patent Literature 4: NEDERBERG F ET AL: "New Paradigms for Organic Catalysts: The First Organocatalytic Living Polymerization",ANGEWANDTE CHEMIE, VERLAG CHEMIE, HOBOKEN, USA, vol. 40, no. 14, 10 July 2001 (2001-07-10), pages 2712-2715, XP002638879,ISSN: 1433-7851

### Summary of Invention

### Technical Problem

A main objective of the present invention is to provide a method for producing a novel lactide complex using a lactide.

### Solution to Problem

The method for producing a lactide complex according to an embodiment of the present invention uses a lactide which is a cyclic dimer of lactic acid, and comprises a reaction step of heating a mixture of the lactide in a molten state with a low-molecular-weight substance to react the molten lactide with the low-molecular-weight substance without ring-opening of the lactide, wherein the molten lactide is obtained by melting any of L-lactide, D-lactide, and DL-lactide, the low-molecular-weight substance has a molecular weight of 2000 or less, and the mixture is stirred in the reaction step.

The method for producing a lactide complex according to an embodiment comprises a reaction step of heating a mixture of a molten lactide with a low-molecular-weight substance to react the molten lactide with the low-molecular-weight substance without ring-opening of the lactide. Preferably, the production method of this embodiment satisfies any one or more of the following conditions (1) to (9):
(1) the mixture is obtained by melting a solid lactide and adding the low-molecular-weight substance to the molten lactide;
(2) the heating is performed at 80°C or more and 180°C or less;
(3) the reaction step is performed in a reduced pressure environment of 5 mmHg or more and 300 mmHg or less;
(4) the mixture is stirred in the reaction step;
(5) the molten lactide is obtained by melting any of L-lactide, D-lactide, and DL-lactide;
(6) the low-molecular-weight substance has a molecular weight of 2000 or less;
(7) the low-molecular-weight substance has any of a C=O bond, a P=O bond, an N=O bond, an N-H bond, and an S=O bond;
(8) the number of moles of the molten lactide in the mixture is five times or more and 500 times or less the number of moles of the low-molecular-weight substance; and
(9) the method comprises a removal step of removing unreacted lactide and unreacted low-molecular-weight substance with an aqueous solvent after the reaction step.

### Advantageous Effects of Invention

The production methods of the present invention can produce novel lactide complexes using lactides.

### Brief Description of Drawings

[FIG. 1] FIG. 1 is the IR spectrum of a lactide.
[FIG. 2] FIG. 2 is the IR spectrum of a lactic acid polymer.
[FIG. 3] FIG. 3 is the IR spectrum of the lactide complex obtained in Example 1.
[FIG. 4] FIG. 4 is the IR spectrum of the lactide complex obtained in Example 2.
[FIG. 5] FIG. 5 is the mass spectrum of a lactide.
[FIG. 6] FIGS. 6A and 6B are the mass spectra of a lactic acid polymer.
[FIG. 7] FIGS. 7A and 7B are the mass spectra of the lactide complex obtained in Example 1.
[FIG. 8] FIGS. 8A and 8B are the mass spectra of the lactide complex obtained in Example 2.
[FIG. 9] FIG. 9 is an illustration showing an example of an apparatus for producing a lactide complex.

### Description of Embodiments

### <<Method for producing lactide complex>>

The method for producing a lactide complex according to an embodiment of the present invention (hereinafter called "the production method according to an embodiment") will be described below. The production method according to an embodiment comprises a reaction step of heating a mixture of a molten lactide with a low-molecular-weight substance to react the molten lactide with the low-molecular-weight substance without ring-opening of the lactide. The production method according to an embodiment can produce novel lactide complexes.

Although the structure of a lactide complex produced by the production method according to an embodiment is not necessarily clear at present, the measurement results from the IR and mass spectra as described below suggest that the lactide complex produced has multiple lactide molecules interacting with and multiply bonded to each other through a low-molecular-weight substance.

### (Lactide)

Lactides used in the production method according to an embodiment are not limited, and conventionally known lactides may be appropriately selected and used. In the present disclosure, the term "lactide" refers to a cyclic dimer of lactic acid.

Lactides used in the production method according to an embodiment are any of L-lactide, D-lactide, and DL-lactide. L-lactide has a melting point of about 94°C to 96°C. D-lactide has a melting point of about 95°C to 98°C. DL-lactide has a melting point of about 116°C to 124°C.

Either one kind or two or more kinds of lactides may be used in the production method according to an embodiment. For example, any one of D-lactide, L-lactide, and DL-lactide may be used, or a combination of two or more kinds of such lactides may be used.

Examples of molten lactides may include molten D-lactide and molten L-lactide. Any one of molten D-lactide and molten L-lactide may be used, or both of them may be used, in the production method according to an embodiment. For example, when DL-lactide is used or a combination of D- and L-lactides is used, molten lactides include both molten D-lactide and molten L-lactide.

The production method according to an embodiment comprises a reaction step of heating a mixture of at least one molten lactide, obtained by melting at least one of D-lactide, L-lactide, and DL-lactide, with a low-molecular-weight substance to react the molten lactide with the low-molecular-weight substance without ring-opening of the lactide.

The production method according to an embodiment comprises a reaction step of heating a mixture of any one or both of molten D-lactide and molten L-lactide with a low-molecular-weight substance to react the molten lactide with the low-molecular-weight substance without ring-opening of the lactide.

### (Low-molecular-weight substance)

Low-molecular-weight substances used in the production method according to an embodiment are not limited and may be appropriately selected depending on the applications of the lactide complexes produced. Either one kind or two or more kinds of low-molecular-weight substances may be used in the production method according to an embodiment.

The molecular weight of a low-molecular-weight substance is not limited, and an exemplary low-molecular-weight substance has a molecular weight of 2000 or less. An exemplary low-molecular-weight substance has a molecular weight of 10 or more. Another exemplary low-molecular-weight substance has a molecular weight of 1500 or less. Another exemplary low-molecular-weight substance has a molecular weight of 100 or more. The molecular weight in the present disclosure refers to number average molecular weight (Mn). A low-molecular-weight substance may have a molecular weight of more than 2000. The molecular weight should be more than 0 and may be less than 10.

Physiologically active substances are exemplary low-molecular-weight substances. Examples of physiologically active substances may include vitamins, amino acids, hydroxy acids, steroid hormones, and amino acid derivative hormones.

Examples of such physiologically active substances may include retinol, thiamine, thiamine phosphate, riboflavin, niacin, pantothenic acid, pantetheine, pyridoxal, pyridoxal phosphate, pyridoxamine, pyridoxine, biotin, folic acid, tetrahydrofolic acid, cyanocobalamin, methylcobalamin, hydroxocobalamin, ascorbic acid, sodium ascorbyl phosphate, magnesium ascorbyl phosphate, ergocalciferol, cholecalciferol, tocopherol, tocotrienol, salicylic acid, calcium salicylate, citric acid, sodium citrate, glutamine, glutamic acid, sodium glutamate, alanine, arginine, asparagine, aspartic acid, cysteine, glycine, histidine, isoleucine, leucine, lysine, methionine, phenylalanine, proline, serine, threonine, tryptophan, tyrosine, valine, malic acid, citric acid, cortisol, androgen, adrenaline, dopamine, acetylcholine, and thyroxine.

Low-molecular-weight substances used in the fields of reagents, synthetic raw materials, pharmaceutical products, agricultural chemicals, cosmetics, foods, functional foods, food additives, feeds, and the like are exemplary low-molecular-weight substances.

Low-molecular-weight substances used may be soluble in water, or may be insoluble or scarcely soluble in water. The production method according to an embodiment can produce lactide complexes insoluble or scarcely soluble in water while using low-molecular-weight substances soluble in water. Accordingly, low-molecular-weight substances can be suitably used which are soluble in water but are intended for use to provide substances insoluble or scarcely soluble in water.

In the present disclosure, the term "low-molecular-weight substance soluble in water" refers to such a substance that 0.1 g of the low-molecular-weight substance can be dissolved in 100 ml of water and precipitation of the low-molecular-weight substance cannot be observed after the passage of three hours.

Exemplary low-molecular-weight substances have any of a C=O bond (or a C=O group), a P=O bond (or a P=O group), an N=O bond (or an N=O group), an N-H bond (or an N-H group), and an S=O bond (or an S=O group). Low-molecular-weight substances having any of such bonds are highly reactive with lactides.

The thermal decomposition temperature of a low-molecular-weight substance may be higher than, lower than, or equal to the melting point of a lactide.

The thermal decomposition temperature of an exemplary low-molecular-weight substance is higher than the melting point of a lactide. In the reaction step, a mixture of the molten lactide with the low-molecular-weight substance is heated at a temperature lower than the thermal decomposition temperature of the low-molecular-weight substance. A mixture of the molten lactide with the low-molecular-weight substance may also be heated at a temperature equal to or higher than the thermal decomposition temperature of the low-molecular-weight substance.

Low-molecular-weight substances may be used as solids or as solutions or dispersions in appropriate solvents.

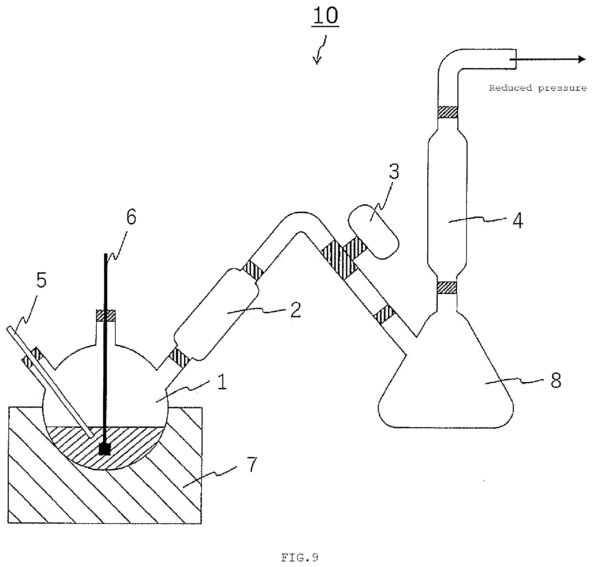

FIG. 9 is an example of a production apparatus 10 that can be used for the production method according to an embodiment. The production apparatus 10 in the illustrated embodiment includes a reaction vessel 1, an air-cooling tube 2, a vacuum gauge 3, a water-cooling tube 4, a thermometer 5, a stirring method (stirring bar) 6, a heating method 7, and a collection vessel 8. An exemplary production apparatus has a pressure reducing method. An exemplary collection vessel 8 collects a lactide that can be sublimed by heating.

In the production method according to an embodiment, a solid lactide (which may be crystalline) is introduced into the reaction vessel 1, and the solid lactide is molten before introducing a low-molecular-weight substance. This embodiment can increase the yield of the lactide complex produced. The lactide can be molten by heating the reaction vessel 1 at a temperature higher than the melting point of the lactide. The reaction vessel 1 can be heated by various heating methods appropriately selected. Examples of the heating methods may include various heaters such as mantle heaters. For example, the reaction vessel 1 is heated by a combination of an oil bath with a heater. The low-molecular-weight substance is then added to the molten lactide. Thus, a mixture of the molten lactide with the low-molecular-weight substance is obtained. Preferably, the low-molecular-weight substance is added while stirring the molten lactide. This embodiment can further increase the yield of the lactide complex produced.

An exemplary mixture is obtained by mixing a solid lactide with a low-molecular-weight substance and then melting the solid lactide.

Hereinafter, the term "mixture" refers to a mixture of a molten lactide with a low-molecular-weight substance.

The amounts of the molten lactide and the low-molecular-weight substance added based on the total mass of the mixture are not limited, and are preferably adjusted so that the number of moles of the molten lactide is five times or more and 500 times or less the number of moles of the low-molecular-weight substance. Such addition can ensure sufficient reaction between the molten lactide and the low-molecular-weight substance and can further increase the yield of the lactide complex produced.

The low-molecular-weight substance used is preferably such a substance that 0.1 g of the low-molecular-weight substance is dissolved in 100 ml of water to provide an aqueous solution having a pH of 4 or more and 9 or less. Use of such a low-molecular-weight substance can further increase the yield of the lactide complex produced. An acid may be added to a functional group of such a low-molecular-weight substance so that 0.1 g of the low-molecular-weight substance is dissolved in 100 ml of water to provide an aqueous solution having a pH of 4 or more and 9 or less. Examples of such an acid may include hydrochloric acid, free carbonic acid, phosphoric acid, nitric acid, and sulfuric acid. A metal salt may also be provided by replacing a hydrogen atom of such an acid with a metal ion. Examples of the metal ion may include sodium, potassium, and calcium ions. The low-molecular-weight substance may also be a derivative having an ester or ether bond or the like.

### <Reaction step>

The reaction step is a step of heating a mixture of a molten lactide with a low-molecular-weight substance to react the molten lactide with the low-molecular-weight substance without ring-opening of the lactide. A lactide complex in which a lactide is reacted with a low-molecular-weight substance can be produced through this step.

In the production method according to an embodiment, a molten lactide is reacted with a low-molecular-weight substance without ring-opening of the lactide. There are no limitations to the method of reacting a molten lactide with a low-molecular-weight substance without ring-opening of the lactide, unless various conventionally known methods that allow ring-opening or ring-opening polymerization of lactides are used. For example, the mixture can be heated without using various ring-opening polymerization catalysts or ring-opening polymerization initiators. Low-molecular-weight substances that do not function to allow ring-opening of lactides can be selected as substances to be reacted with molten lactides. Components that can allow ring-opening of lactides are not used in an exemplary production method.

Lactides used in the production method according to an embodiment are easily decomposed by water, characteristically, but the lactide complexes produced are not decomposed by water and are insoluble or scarcely soluble in water.

Although the mechanism to make lactide complexes insoluble or scarcely soluble in water is currently unclear, it is also clear from the results of Examples described below that lactide complexes are insoluble or scarcely soluble in water while having components derived from lactides and low-molecular-weight substances.

Heating in the reaction step is preferably performed while stirring the mixture. This embodiment can further increase the yield of the lactide complex produced.

Although the mixture in the reaction vessel 1 is mixed by the stirring bar 6 in the illustrated embodiment, stirring may also be performed by other methods. Examples of other stirring methods may include magnetic stirrers.

The speed of stirring the mixture is preferably 50 rpm or more, and more preferably 150 rpm or more and 500 rpm or less.

The heating temperature in the reaction step is not limited, and is preferably 180°C or less. This embodiment can turn the produced lactide complex into powder easily.

The lower limit of the heating temperature is not limited, only if the lactide is maintained in a molten state during the reaction. In the reaction step, a mixture may be heated at the melting point of the lactide or at a higher temperature, or may be heated at a temperature lower than the melting point of the lactide when freezing point depression occurs due to mixing of the molten lactide with the low-molecular-weight substance. The heating temperature of the mixture is 80°C or more and 180°C or less, for example.

Although the temperature of the mixture is measured with a contact-type thermometer in the illustrated embodiment, the temperature of the mixture may also be measured with a non-contact-type thermometer.

The heating time is not limited and may be appropriately determined according to the low-molecular-weight substance used. In the production method according to an embodiment, the mixture is heated at the heating temperature described above for 0.5 hours or more and 8 hours or less.

The reaction step may be performed in a normal pressure environment or in a reduced pressure environment. The reaction step in a preferred embodiment is performed in a reduced pressure environment.

The reaction step in a more preferred embodiment is performed in a reduced pressure environment of 5 mmHg or more and 300 mmHg or less. This embodiment can further increase the yield of the lactide complex produced. It can also suppress the loss when the lactide that can be partially sublimed by heating is collected in the collection vessel 8.

The pressure in the reaction vessel 1 is reduced by a pressure reducing method in the illustrated embodiment. Examples of the pressure reducing method may include vacuum pumps and aspirators. The pressure in the reaction vessel 1 may also be reduced by other methods.

### <Removal step>

The production method according to an embodiment may comprise a removal step of removing the unreacted lactide and the unreacted low-molecular-weight substance after the reaction step.

In an exemplary removal step, an aqueous solvent is added to the reaction vessel 1 after the reaction step. Examples of the aqueous solvent may include water and alcohols.

Because lactides are easily decomposed in water, characteristically, the lactide and the low-molecular-weight substance can be dissolved in an aqueous solvent in the production method according to an embodiment when a low-molecular-weight substance soluble in water is used.

On the other hand, the lactide complex produced cannot be dissolved in an aqueous solvent and is present as a precipitate or suspended matter in the reaction vessel 1, because it is insoluble or scarcely soluble in water. Accordingly, the unreacted lactide and the unreacted low-molecular-weight substance can be removed by discharging from the reaction vessel 1 the aqueous solvent in which the unreacted lactide and the unreacted low-molecular-weight substance are dissolved.

In sum, removal of the unreacted lactide and the unreacted low-molecular-weight substance by an aqueous solvent 1 is an optional step in the production method according to an embodiment. Properties of the lactide complex produced are not changed after the addition of the aqueous solvent 1.

Preferably, the removal step is performed after the temperature of the lactide complex produced becomes 100°C or less following the reaction step.

In an exemplary removal step, an alcohol in an amount about 1 to 5 times the amount of the lactide used is added after the reaction step into the reaction vessel 1 containing the produced lactide complex, the unreacted lactide, and the unreacted low-molecular-weight substance (hereinafter these substances are collectively called " assembly "), so that a assembly solution containing the assembly is provided. Examples of the alcohol may include methyl alcohol and ethyl alcohol.

The assembly solution is taken from the reaction vessel 1. The assembly solution is gradually added to an amount of water 10 times or more the amount of the assembly solution with stirring, and the stirring is performed for 24 hours or more.

The unreacted lactide and the unreacted low-molecular-weight substance can be removed while replacing water every three to four hours.

In another exemplary removal step, a assembly solution obtained in the same manner as described above is enclosed in a dialysis tube or the like and dialyzed with running water for five days or more to remove the unreacted lactide and the unreacted low-molecular-weight substance. Examples of the dialysis tube may include cellulose tubes.

### <Purification step>

The production method according to an embodiment may comprise a purification step of purifying the produced lactide complex after the reaction step.

In an exemplary purification step, the lactide complex obtained as a precipitate or suspended matter is filtered or centrifuged and then dried by various drying methods to purify the lactide complex. Examples of the drying methods may include heat drying, freeze drying, and spray drying.

An exemplary purified lactide complex is powdered, oily, or glassy.

A preferred method for producing a lactide complex according to an embodiment uses a lactide which is a cyclic dimer of lactic acid, and comprises a reaction step of heating a mixture of the lactide in a molten state with a low-molecular-weight substance to react the molten lactide with the low-molecular-weight substance without ring-opening of the lactide, wherein the molten lactide is obtained by melting any one or more of L-lactide, D-lactide, and DL-lactide, the low-molecular-weight substance has a molecular weight of 2000 or less, and the mixture is stirred in the reaction step.

A preferred method for producing a lactide complex according to an embodiment uses a lactide which is a cyclic dimer of lactic acid, and comprises a reaction step of heating a mixture of any one or both of molten D-lactide and molten L-lactide with a low-molecular-weight substance to react the molten lactide with the low-molecular-weight substance without ring-opening of the lactide, wherein the low-molecular-weight substance has a molecular weight of 2000 or less, and the mixture is stirred in the reaction step.

### Examples

Next, a method for producing lactide a complex will be described by way of examples.

### (Example 1)

300 g of D-lactide was introduced into a reaction vessel, and the reaction vessel was heated at 116°C to melt the D-lactide. With the reaction vessel kept heated, 35.5 g of powdered pyridoxine hydrochloride was gradually added over two minutes while stirring the molten D-lactide at 300 rpm to provide a mixture of the D-lactide with pyridoxine hydrochloride.

The pressure in the reaction vessel was reduced to 30 mmHg, and the reaction vessel was heated for four hours while stirring the mixture at 250 rpm with the heating temperature controlled at 135°C to 145°C to react the D-lactide with pyridoxine hydrochloride.

After completion of the reaction, the mixture was left to stand at room temperature for 15 minutes, and 500 ml of warmed methanol was then added into the reaction vessel to provide a suspension. The suspension was placed in a dialysis tube, 300 ml of water was added, and dialysis was performed for 10 days.

The precipitate was collected by centrifugation (1000 rpm x 30 min) using a centrifuge. The precipitate was freeze-dried to provide a lactide complex of Example 1. The lactide complex of Example 1 is a complex of D-lactide with pyridoxine hydrochloride.

The mass of the resulting lactide complex was 179.42 g.

### (Example 2)

300 g of D-lactide was introduced into a reaction vessel, and the reaction vessel was heated at 122°C to melt the D-lactide. With the reaction vessel kept heated, 20.1 g of powdered sodium L-malate was gradually added over two minutes while stirring the molten D-lactide at 300 rpm to provide a mixture of the D-lactide with sodium L-malate.

The pressure in the reaction vessel was reduced to 30 mmHg, and the reaction vessel was heated for four hours while stirring the mixture at 250 rpm with the heating temperature controlled at 130°C to 140°C to react the D-lactide with sodium L-malate.

After completion of the reaction, the mixture was left to stand at room temperature for 15 minutes, and 500 ml of warmed methyl alcohol was then added into the reaction vessel to provide a suspension. The suspension was placed in a dialysis tube, 300 ml of water was added, and dialysis was performed for 10 days.

The precipitate was collected by centrifugation (1000 rpm x 30 min) using a centrifuge. The precipitate was freeze-dried to provide a lactide complex of Example 2. The lactide complex of Example 2 is a complex of D-lactide with sodium L-malate.

The mass of the resulting lactide complex was 167.1 g.

### (Measurement of IR spectra)

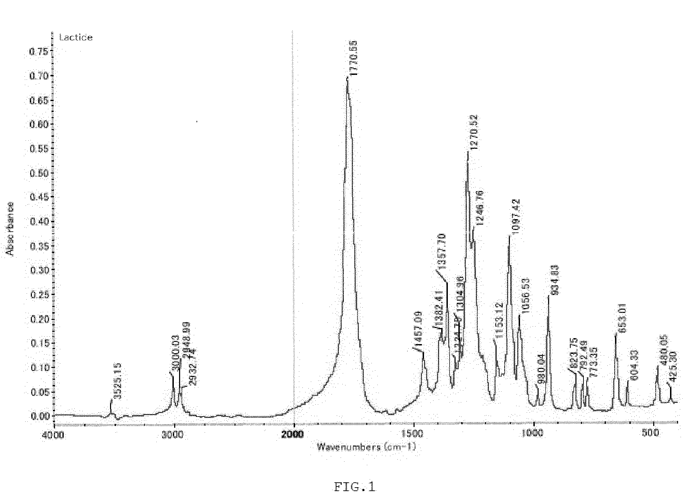

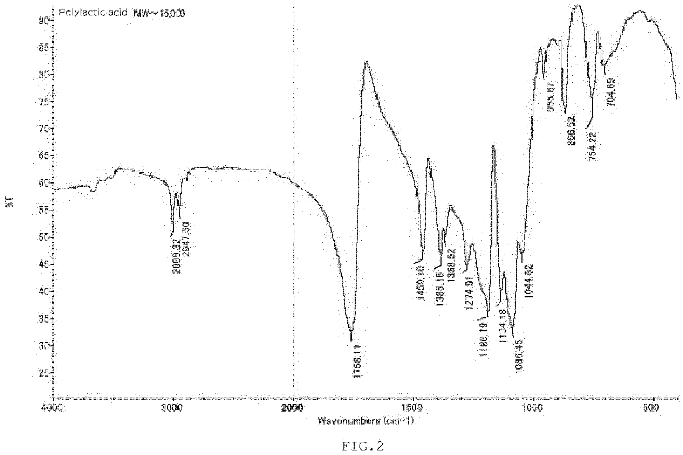

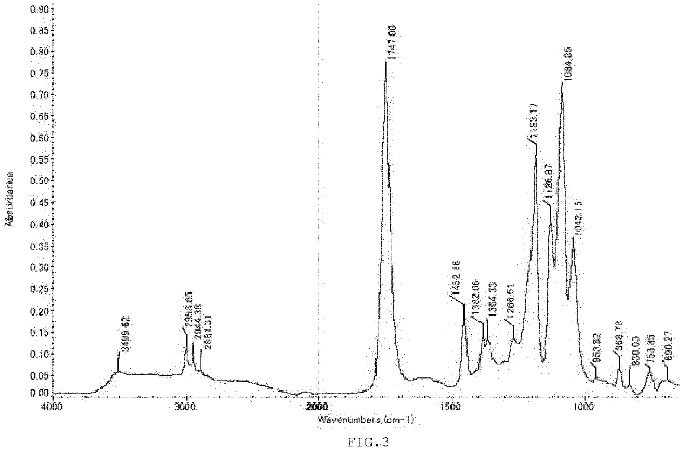

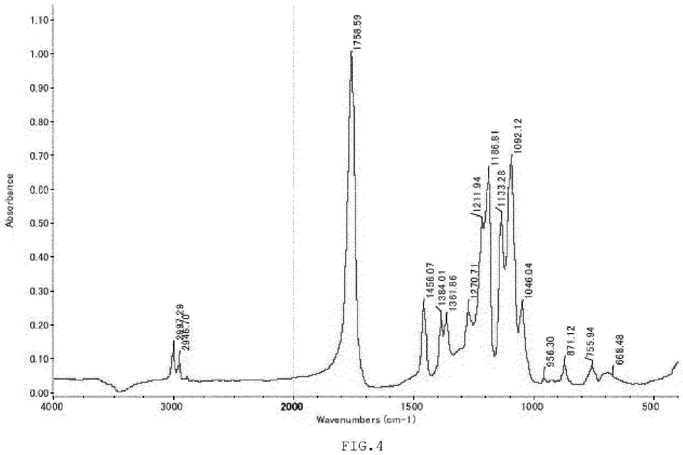

The IR spectra of a lactide, a lactic acid polymer, and the lactide complex of Example 2 were measured under the following measurement condition 1, and the IR spectrum of the lactide complex of Example 1 was measured under the following measurement condition 2. The measurement results are shown in FIGS. 1 to 4. FIG. 1 is the IR spectrum of the lactide, FIG. 2 is the IR spectrum of the lactic acid polymer, FIG. 3 is the IR spectrum of the lactide complex obtained in Example 1, and FIG. 4 is the IR spectrum of the lactide complex obtained in Example 2.

### (Measurement condition 1)

Test method: Infrared spectroscopy (FT-IR)
Pretreatment: KBr tablet method (Cryopreserved samples were brought back to normal temperature and then crushed with a mortar, and KBr tablets were formed and tested.)
Measurement method: Transmission
Measurement range: 4000 cm⁻¹ to 400 cm⁻¹
Resolution: 4 cm⁻¹
Cumulative number: 40
Apparatus used: Nicolet 8700 FT-IR (manufactured by Thermo Fisher Scientific)

### (Measurement condition 2)

Test method: Infrared spectroscopy (FT-IR)
Measurement method: ATR method (ATR crystals: ZnSe)
Measurement range: 4000 cm⁻¹ to 650 cm⁻¹
Resolution: 4 cm⁻¹
Cumulative number: 40
Apparatus used: Nicolet 8700 FT-IR (manufactured by Thermo Fisher Scientific)

### (Measurement of mass spectra)

Solvents were added to a lactide, a lactic acid polymer, the lactide complex of Example 1, and the lactide complex of Example 2 to dissolve them, and their mass spectra were then measured under the following measurement condition 3. The solvent used for dissolution of the lactide was dimethyl sulfoxide, the solvent used for dissolution of the lactic acid polymer and the lactide complex of Example 2 was acetone, and the solvent used for dissolution of the lactide complex of Example 1 was methanol.

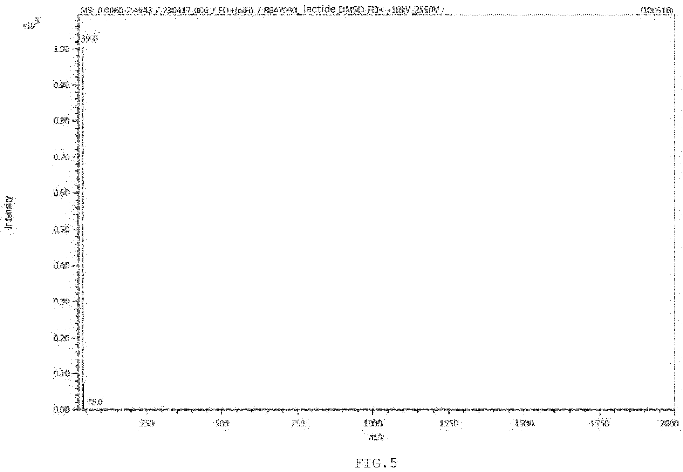

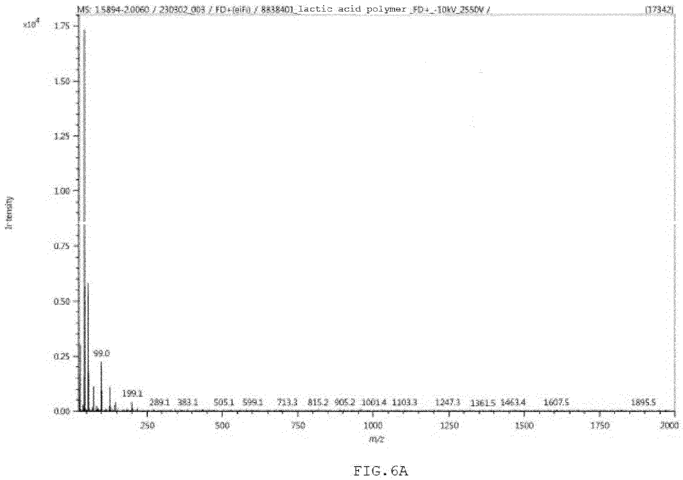

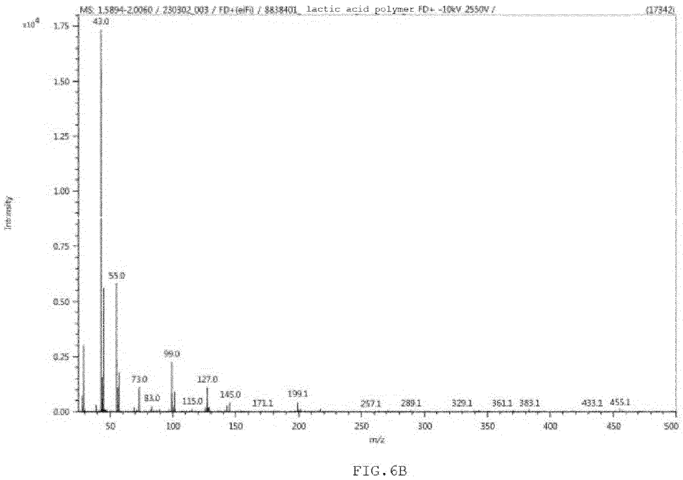

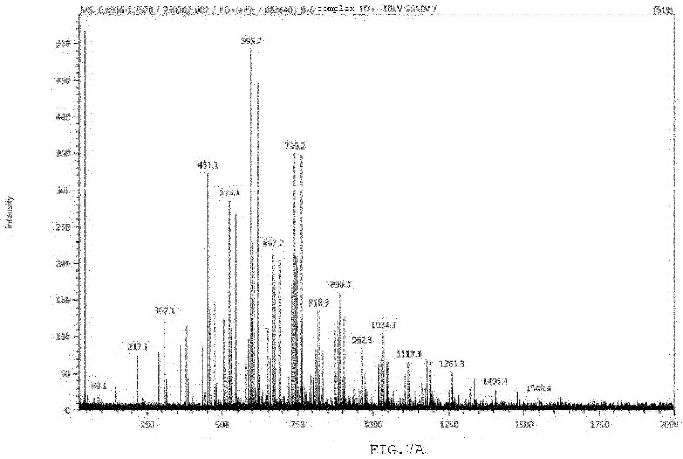

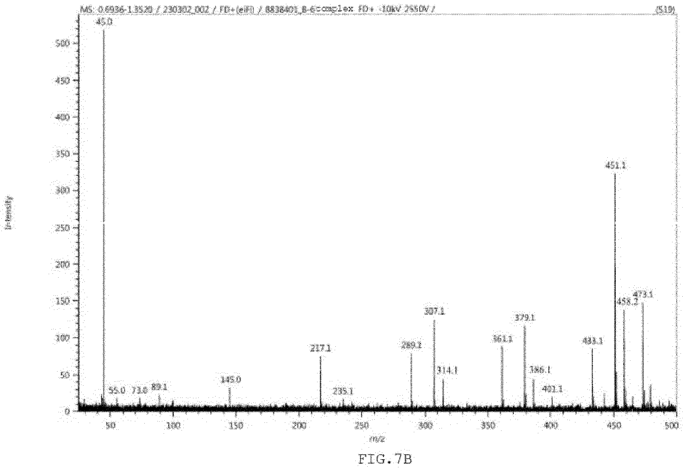

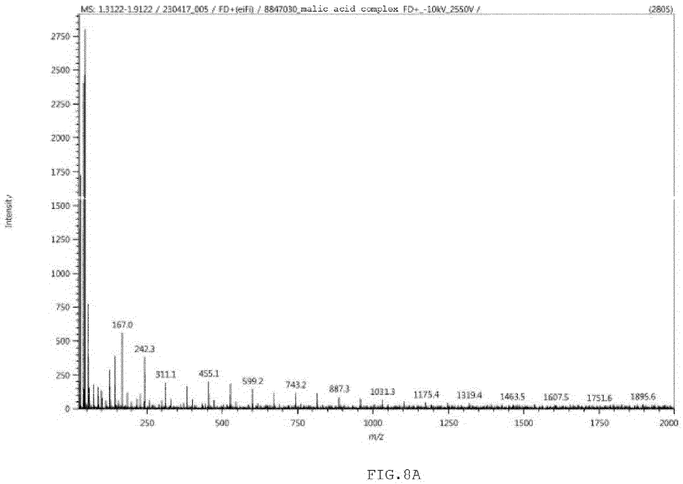

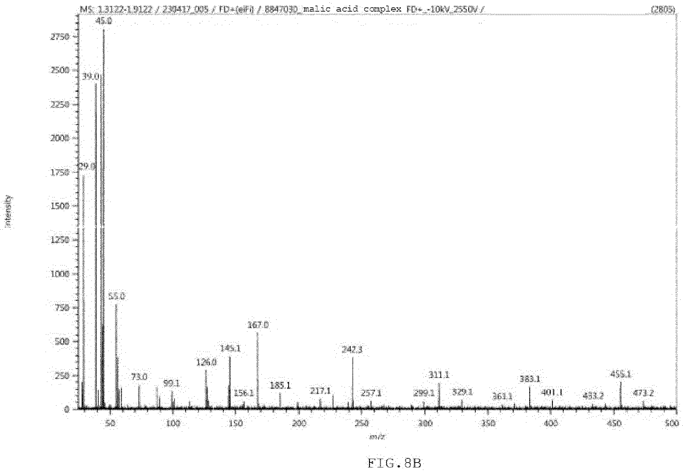

The measurement results of mass spectra are shown in FIGS. 5 to 8. FIG. 5 is the mass spectrum of the lactide, FIGS. 6A and 6B are the mass spectra of the lactic acid polymer, FIGS. 7A and 7B are the mass spectra of the lactide complex obtained in Example 1, and FIGS. 8A and 8B are the FD mass spectra of the lactide complex obtained in Example 2.

### (Measurement condition 3)

Test method: FD/MS
Apparatus: JMS-T200GC mass spectrometer (manufactured by JEOL Ltd.)
Ionization method: FD(+)
Counter electrode: -10 kV
Emitter: Carbon

### Measured scan range: m/z 25 to 2000

The specific absorption bands in the IR spectrum of the lactide are 1770 cm⁻¹, 1270 cm⁻¹, and 1097 cm⁻¹ as shown in FIG. 1, while the specific absorption bands in the IR spectrum of the lactic acid polymer are 1758 cm⁻¹, 1186 cm⁻¹, and 1086 cm⁻¹ as shown in FIG. 2. The specific absorption bands are distinctly different between both substances. Here, if the lactide complexes of Examples 1 and 2 are lactic acid polymers in which lactides are ring-opened, lactides in a free state, or substances on which lactides are simply adsorbed, the specific absorption bands in the IR spectra of the lactide complexes of Examples 1 and 2 should correspond to the specific absorption bands of the lactide or the lactic acid polymer completely. In this context, the specific absorption bands in the IR spectrum of the lactide complex of Example 1 are 1747 cm⁻¹, 1183 cm⁻¹, and 1085 cm⁻¹ and the specific absorption bands in the IR spectrum of the lactide complex of Example 2 are 1759 cm⁻¹, 1187 cm⁻¹, and 1092 cm⁻¹, as shown in FIGS. 3 and 4. These specific absorption bands are obviously different from the specific absorption bands of the lactide and the lactic acid polymer. These absorption bands are also observed in the differential spectra of the lactide complexes and the low-molecular-weight compound. As is clear from these measurement results, the lactide complexes of Examples 1 and 2 are not lactic acid polymers in which lactides are ring-opened, lactides in a free state, and substances on which lactides are simply adsorbed.

The specific fragment in the mass spectrum of the lactide is 39 as shown in FIG. 5, while the specific fragment in the mass spectra of the lactic acid polymer is 43 as shown in FIGS. 6A and 6B. The specific fragments are distinctly different between both substances. Here, as shown in FIGS. 7A and 7B, in the lactide complex of Example 1 using, as a low-molecular-weight compound, pyridoxine hydrochloride of which the mass spectrum can be measured, a specific fragment can be observed at 45 and a mountain-like fragment assembly can be observed at 300 to 1300. Many fragments at intervals of 144 each, corresponding to the molecular weight of the lactide, 144, can be observed in this mountain-like fragment assembly. On the other hand, the specific fragment 39 of the lactide and the specific fragment 43 of the lactic acid polymer cannot be observed in the lactide complex of Example 1.

As shown in FIGS. 8A and 8B, in the lactide complex of Example 2 using, as a low-molecular-weight substance, metal salt-containing sodium L-malate of which the mass spectrum cannot be measured, the specific fragment 39 of the lactide, the specific fragment 43 of the lactic acid polymer, and the same specific fragment 45 as that of the lactide complex of Example 1 can all be observed. Measurement of the mass spectrum of the lactide complex of Example 2 suggests that, due to the measurement environment (high temperature, high vacuum), the complex of D-lactide with sodium L-malate is separated and decomposed, and the multiply bonded lactide is isolated and reacted again under high temperature and high vacuum conditions, so that the same specific fragments as those of the lactide, the multiply bonded lactide, and the lactic acid polymer are mixed together. In this context, presumably, if the lactide complex of Example 2 is a complex obtained by ring-opening polymerization of the lactide, only the specific fragment 43 of the lactic acid polymer would be measured, or sodium L-malate could not be measured due to the presence of the metal salt.

As is clear from the measurement results of the IR and mass spectra above, the lactide complexes of Examples 1 and 2 are complexes in which lactides are reacted with low-molecular-weight substances while maintaining their cyclic structures.

Next, production examples for lactide complexes using D-lactide, L-lactide, and DL-lactide will be described by way of Reference Examples 1 to 5. Reference Example A is a comparative production example in which a lactide complex cannot be produced.

Reference Example 1 is a production example for a lactide complex in which D-lactide (molten D-lactide) is reacted with a low-molecular-weight substance.

Reference Examples 2 to 4 are production examples for a lactide complex in which L-lactide (molten L-lactide) is reacted with a low-molecular-weight substance.

Reference Example 5 is a production example for a lactide complex in which DL-lactide (molten DL-lactide) is reacted with a low-molecular-weight substance.

Reference Example A is a production example in which D-lactide is not molten.

### (Reference Example 1)

225.12 g of D-lactide was introduced into a reaction vessel, and the reaction vessel was heated at 120°C to melt the D-lactide. With the reaction vessel kept heated, 14.9 g of powdered sodium salicylate was gradually added over two minutes while stirring the molten D-lactide at 250 rpm to provide a mixture of the D-lactide with sodium salicylate. The pressure in the reaction vessel was reduced to 30 mmHg, and the reaction vessel was heated for four hours while stirring the mixture at 250 rpm with the heating temperature controlled at 125°C to 135°C to react the D-lactide with sodium salicylate. After completion of the reaction, the mixture was left to stand at room temperature for 15 minutes, and 600 ml of methyl alcohol was then added into the reaction vessel to provide a suspension. The suspension was placed in a dialysis tube, 600 ml of water was added, and dialysis was performed for 7 days. A precipitate which is a lactide complex could be collected by centrifugation (500 rpm x 30 min) using a centrifuge. The precipitate was freeze-dried to provide 186.89 g of a D-lactide-sodium salicylate complex.

### (Reference Example 2)

300 g of L-lactide was introduced into a reaction vessel, and the reaction vessel was heated to 117°C to melt the L-lactide. With the reaction vessel kept heated, 17.01 g of powdered water-soluble L-ascorbic acid 2-phosphate trisodium salt (molecular weight: 322.05) was gradually added over two minutes while stirring the molten L-lactide at 300 rpm to provide a mixture of the L-lactide with L-ascorbic acid 2-phosphate trisodium salt. The pressure in the reaction vessel was reduced to 30 mmHg, and the reaction vessel was heated for four hours while stirring the mixture at 250 rpm with the heating temperature controlled at 105°C to 115°C to react the L-lactide with L-ascorbic acid 2-phosphate trisodium salt. After completion of the reaction, the mixture was left to stand at room temperature for 10 minutes, and 500 ml of methyl alcohol was then added into the reaction vessel while stirring at 300 rpm to provide a suspension. The suspension was placed in a dialysis tube, 50 ml of water was added, and dialysis was performed for 10 days. A precipitate which is a lactide complex could be collected by centrifugation (1000 rpm x 30 min) using a centrifuge. The precipitate was freeze-dried to provide 148 g of an L-lactide-L-ascorbic acid 2-phosphate trisodium salt complex.

### (Reference Example 3)

200 g of L-lactide was blended with 10.08 g of water-soluble and hygroscopic acetylcholine chloride (molecular weight: 181.66), and the mixture was made into fine powder with a mortar. The mixture was introduced into a reaction vessel, and the reaction vessel was heated to 115°C while stirring the mixture at 300 rpm to melt the mixture. The pressure in the reaction vessel was then reduced to 30 mmHg, and the reaction vessel was heated for four hours while stirring the mixture (molten mixture) at 250 rpm with the heating temperature controlled at 130 to 135°C to react the L-lactide with acetylcholine chloride. After completion of the reaction, the mixture was left to stand at room temperature for 10 minutes while stirring at 250 rpm, and 400 ml of methanol warmed to 65°C was then added into the reaction vessel while stirring at 300 rpm to provide a methanol solution. The methanol solution was placed in a dialysis tube (cellulose tube), 50 ml of water was added, and dialysis was performed in running water for 14 days. The solid generated in the solution in the dialysis tube was freeze-dried to provide 64.89 g of an L-lactide-acetylcholine complex.

### (Reference Example 4)

300 g of L-lactide was introduced into a reaction vessel, and the reaction vessel was heated to 100°C to melt the L-lactide. With the reaction vessel maintained at 111°C, 22.85 g of fat-soluble (scarcely water-soluble) vitamin A acetate (molecular weight: 328.49) (fine powder) was gradually added over two minutes while stirring the molten L-lactide at 300 rpm to provide a mixture of the L-lactide with vitamin A acetate. The pressure in the reaction vessel was then reduced to 30 mmHg, and the reaction vessel was heated for 3.5 hours while stirring the mixture at 250 rpm with the heating temperature controlled at 130 to 135°C to react the L-lactide with vitamin A acetate. After completion of the reaction, the mixture was left to stand at room temperature for 15 minutes while stirring at 250 rpm, and 500 ml of methanol warmed to 65°C was then added into the reaction vessel while stirring at 300 rpm to provide a methanol solution. The methanol solution was placed in a dialysis tube (cellulose tube), 50 ml of water was added, and dialysis was performed in running water for 14 days. The solid generated in the solution in the dialysis tube was freeze-dried to provide 145.56 g of an L-lactide-vitamin A acetate complex.

### (Reference Example 5)

275 g of DL-lactide was introduced into a reaction vessel, and the reaction vessel was heated to 126°C to melt the DL-lactide. With the reaction vessel kept heated, 8 g of powdered water-soluble cyanocobalamin (molecular weight: 1355.37) was gradually added over two minutes while stirring the molten DL-lactide at 300 rpm to provide a mixture of the DL-lactide with cyanocobalamin. The pressure in the reaction vessel was reduced to 30 mmHg, and the reaction vessel was heated for four hours while stirring the mixture at 250 rpm with the heating temperature controlled at 145°C to 150°C to react the DL-lactide with cyanocobalamin. After completion of the reaction, the mixture was left to stand at room temperature for 20 minutes, and 500 ml of methyl alcohol was then added into the reaction vessel while stirring at 300 rpm to provide a suspension. The suspension was placed in a dialysis tube, 50 ml of water was added, and dialysis was performed for 14 days. The solid generated in the solution in the dialysis tube was taken out and freeze-dried to provide 60.44 g of a DL-lactide-cyanocobalamin complex.

### (Reference Example A)

22.5 g of D-lactide and 1.5 g of sodium salicylate were introduced into a mortar and sufficiently crushed and blended to provide a powder mixture of the D-lactide with sodium salicylate. The powder mixture was placed in a beaker and allowed to stand for four hours in a desiccator at room temperature and in a reduced pressure environment of 30 mmHg. Thereafter, 50 ml of methyl alcohol was added to the powder mixture to provide a solution. The solution was placed in a dialysis tube, 50 ml of water was added, and dialysis was performed for 7 days. No suspended matter could be observed in the dialysis tube. The solution in the dialysis tube was placed in a flask, and water was removed under reduced pressure. No residue could be observed in the flask.

In Reference Examples 1 to 5 in which lactides were molten, remaining precipitates (or solids) undissolved in water were observed to be generated, and the amount of the remaining precipitates (or solids) was larger than the amount of the raw material low-molecular-weight substances. On the other hand, in Reference Example A in which a lactide was not molten, no remaining precipitate undissolved in water could be observed. As is also clear from these results, lactide complexes can be produced by melting lactides and reacting the lactides with low-molecular-weight substances.

### Reference Signs List

- 1: Reaction vessel
- 2: Air-cooling tube
- 3: Vacuum gauge
- 4: Water-cooling tube
- 5: Thermometer
- 6: Stirring method
- 7: Heating method
- 8: Collection vessel
- 10: Lactide production apparatus

## Claims

1. A method for producing a lactide complex,
using a lactide which is a cyclic dimer of lactic acid,
the method comprising a reaction step of heating a mixture of the lactide in a molten state with a low-molecular-weight substance to react the molten lactide with the low-molecular-weight substance without ring-opening of the lactide,
wherein the molten lactide is obtained by melting any of L-lactide, D-lactide, and DL-lactide,
the low-molecular-weight substance has a molecular weight of 2000 or less, and
the mixture is stirred in the reaction step.

2. The method for producing a lactide complex according to claim 1, wherein the mixture is obtained by melting a solid lactide and adding the low-molecular-weight substance to the molten lactide.

3. The method for producing a lactide complex according to claim 1 or 2, wherein the heating is performed at 80°C or more and 180°C or less.

4. The method for producing a lactide complex according to claim 1 or 2, wherein the reaction step is performed in a reduced pressure environment of 5 mmHg or more and 300 mmHg or less.

5. The method for producing a lactide complex according to claim 1 or 2, wherein the low-molecular-weight substance has any of a C=O bond, a P=O bond, an N=O bond, an N-H bond, and an S=O bond.

6. The method for producing a lactide complex according to claim 1 or 2, wherein the number of moles of the molten lactide in the mixture is five times or more and 500 times or less the number of moles of the low-molecular-weight substance.

7. The method for producing a lactide complex according to claim 1 or 2, comprising a removal step of removing unreacted lactide and unreacted low-molecular-weight substance with an aqueous solvent after the reaction step.

## Patentansprüche

1. Verfahren zur Herstellung eines Lactidkomplexes,
welches ein Lactid verwendet, das ein cyclisches Dimer von Milchsäure ist,
wobei das Verfahren einen Reaktionsschritt des Erwärmens eines Gemisches des Lactids in einem geschmolzenen Zustand und einer niedermolekularen Substanz umfasst, um das geschmolzene Lactid mit der niedermolekularen Substanz ohne Ringöffnung des Lactids umzusetzen,
wobei das geschmolzene Lactid durch Schmelzen von einem von L-Lactid, D-Lactid oder DL-Lactid erhalten worden ist,
die niedermolekulare Substanz ein Molekulargewicht von 2000 oder weniger aufweist, und
das Gemisch in dem Reaktionsschritt gerührt wird.

2. Verfahren zur Herstellung eines Lactidkomplexes nach Anspruch 1, wobei das Gemisch durch Schmelzen eines festen Lactids und Zugabe der niedermolekularen Substanz zu dem geschmolzenen Lactid erhalten wird.

3. Verfahren zur Herstellung eines Lactidkomplexes nach Anspruch 1 oder 2, wobei das Erwärmen bei 80°C oder mehr und 180°C oder weniger durchgeführt wird.

4. Verfahren zur Herstellung eines Lactidkomplexes nach Anspruch 1 oder 2, wobei der Reaktionsschritt in einer Umgebung mit reduziertem Druck von 5 mmHg oder mehr und 300 mmHg oder weniger durchgeführt wird.

5. Verfahren zur Herstellung eines Lactidkomplexes nach Anspruch 1 oder 2, wobei die niedermolekulare Substanz eine C=O-Bindung, eine P=O-Bindung, eine N=O-Bindung, eine NH-Bindung oder eine S=O-Bindung aufweist.

6. Verfahren zur Herstellung eines Lactidkomplexes nach Anspruch 1 oder 2, wobei die Stoffmenge des geschmolzenen Lactids in dem Gemisch das Fünffache oder mehr und das Fünfhundertfache oder weniger der Stoffmenge der niedermolekularen Substanz beträgt.

7. Verfahren zur Herstellung eines Lactidkomplexes nach Anspruch 1 oder 2, umfassend einen Entfernungsschritt des Entfernens von nicht umgesetztem Lactid und nicht umgesetzter niedermolekularer Substanz mit einem wässrigen Lösungsmittel nach dem Reaktionsschritt.

## Revendications

1. Procédé de production d'un complexe de lactide,
utilisant un lactide qui est un dimère cyclique d'acide lactique,
le procédé comprenant une étape de réaction consistant à chauffer un mélange du lactide dans un état fondu avec une substance à faible poids moléculaire pour faire réagir le lactide fondu avec la substance à faible poids moléculaire sans ouverture de cycle du lactide,
dans lequel le lactide fondu est obtenu en faisant fondre l'un quelconque du L-lactide, D-lactide et DL-lactide,
la substance à faible poids moléculaire a un poids moléculaire de 2000 ou moins, et
le mélange est agité dans l'étape de réaction.

2. Procédé de production d'un complexe de lactide selon la revendication 1, dans lequel le mélange est obtenu en faisant fondre un lactide solide et ajoutant la substance à faible poids moléculaire au lactide fondu.

3. Procédé de production d'un complexe de lactide selon la revendication 1 ou 2, dans lequel le chauffage est réalisé à 80 °C ou plus et 180 °C ou moins.

4. Procédé de production d'un complexe de lactide selon la revendication 1 ou 2, dans lequel l'étape de réaction est réalisée dans un environnement à pression réduite de 5 mmHg ou plus et 300 mmHg ou moins.

5. Procédé de production d'un complexe de lactide selon la revendication 1 ou 2, dans lequel la substance à faible poids moléculaire a l'une quelconque d'une liaison C=O, d'une liaison P=O, d'une liaison N=O, d'une liaison N-H et d'une liaison S=O.

6. Procédé de production d'un complexe de lactide selon la revendication 1 ou 2, dans lequel le nombre de moles du lactide fondu dans le mélange est de cinq fois ou plus et 500 fois ou moins le nombre de moles de la substance à faible poids moléculaire.

7. Procédé de production d'un complexe de lactide selon la revendication 1 ou 2, comprenant une étape d'élimination consistant à éliminer du lactide n'ayant pas réagir et de la substance à faible poids moléculaire n'ayant pas réagi avec un solvant aqueux après l'étape de réaction.
